# EUROPEAN PATENT SPECIFICATION

(11) **EP 0 421 885 B1**
(45) Date of publication and mention of the grant of the patent: **27.12.1995**
(21) Application number: 90402757.0
(22) Date of filing: 04.10.1990
(51) Int. Cl.: C12P 19/04, C12N 1/20

(54) **Bacillus sp. haitai 1 strain, polysaccharide produced by this strain and a process for preparation thereof**
Bacillus sp. Haitai Stamm, ein durch diesen Stamm produziertes Polysaccharid und Verfahren zu dessen Herstellung
Souche de Bacillus sp. haitai, un polysaccharide produit par cette souche et son procédé de préparation

(30) Priority: 04.10.1989 KR 8914213
(43) Date of publication of application: 10.04.1991
(73) Proprietor: HAITAI CONFECTIONERY CO., LTD., Youngdungpo-gu, Seoul (KR); Yu, Ju Hyun, Seodaemoon-gu, Seoul (KR)
(72) Inventor: Yu, Ju Hyun, Seoul (KR); Shin, Yong Mock, Seoul (KR); Rhee, Kyu Soon, Seoul (KR)
(74) Representative: Gillard, Marie-Louise

(56) References cited:
- EP-A- 23 397
- JP-B-43 005 013
- US-A- 3 282 916

## Description

### Field of Invention

The present invention relates to a Bacillus sp. Haitai 1 strain producing polysaccharides which have a thermal stability and can reversibly form a gel by a heat treatment, to polysaccharides having the above characteristics obtainable from that strain and to a process for preparation thereof.

Bacillus sp. Haitai 1 has been deposited in Korean Culture Center of Microorganisms with the accession number of KCCM-10 001.

### Background of the Invention

Polysaccharides take considerable part in the market of aqueous polymers, and have been developed to an industrial application because of their various functional properties. These polysaccharides are present in leaves, stems and roots of plants, seaweeds, animals, microorganisms, mycelium, insect chitin and the like. Natural polysaccharides extracted from plants and seaweeds were almost applicable so far. For example, starch of plants, Locust bean gum and Guar gum extracted from roots of plants, agar and alginate from seaweeds, and pectin from plants. Because the production of natural polysaccharides from plants is variable as regards quantity and quality according to the climate of the year, its costs, quality and supply are irregular and the continuously increasing consumption of natural polysaccharides is not satisfied. Therefore, polysaccharides produced by microorganisms are needed in order to overcome these problems.

Polysaccharides formed by microorganisms are of three types :
a. external polysaccharides of microorganisms presented outside of microorganisms as capsule or slime,
b. internal polysaccharides of microorganisms as energy stock material in microorganisms, and
c. polysaccharides constituting the cell wall.

Recently, due to the commercial success of dextran and xanthan gum among polysaccharides produced by microorganisms, the preparation of such polysaccharide has become of increasing interest. Polysaccharides produced by microorganisms have the following characteristics : an emulsifying capacity, a viscosity increasing capacity, the capacities of stability, gelation and aggregation, a water holding capacity, a film forming capacity and physiological activating capacity. They are thus studied for various uses in the field of foods, cosmetics, oils and medicines. Their use depends on the intrisic properties of the polysaccharide. When the polysaccharides are produced by the fermentation of microorganisms, for example, the quality and quantity of production can be advantageously controlled, the degree of synthesis can be controlled by the fermentation condition, a cheap, plentiful source can be used, a high yield can be obtained by continuous culture and the amount of production can be controlled as to be suitable to a market condition.

Microorganisms producing polysaccharides are known bacteria such as Agrobacter, Alcaligenes, Arthrobacter, Azotobacter, Bacillus, Pseudomonas and Klebsiella, yeast such as Hansenula, Rhodotolura, and must such as Rhizobium and Aspergillus. The studies for the preparation of novel polysaccharides by microorganisms are in progress as only a few of them are applicable in industry, and as polysaccharides having new structures and properties are required.

To produce polysaccharides by a microorganism, a microorganism having subject character is first separated from the soil, culture conditions to produce polysaccharides are then considered with the separated strains, and then the study must be followed about the purification, the stability and application of polysaccharides by culture solution.

### Summary of the Invention

The invention relates to a novel microorganism, Bacillus sp. Haitai 1, deposited at the KCCM under No. 10001, which has been separated from an inland soil.

This novel microorganism produces a viscous polysaccharide which is thermally stable and has the capacities of film forming, water holding, emulsifying and reversibly forming a gel by a heat treatment.

The novel polysaccharide which has the above characters and in which the kind and composition ratio of monosaccharides constituting said polysaccharide are not identical to the known polysaccharides, is a further object of the invention.

The invention also relates to a process for the preparation method of the novel polysaccharide which comprises cultivating Bacillus sp. Haitai 1 in a culture medium comprising a carbon source, a nitrogen source and mineral salts, in particular 2% starch and 1% soybean meal.

### Brief Description of the Drawings.

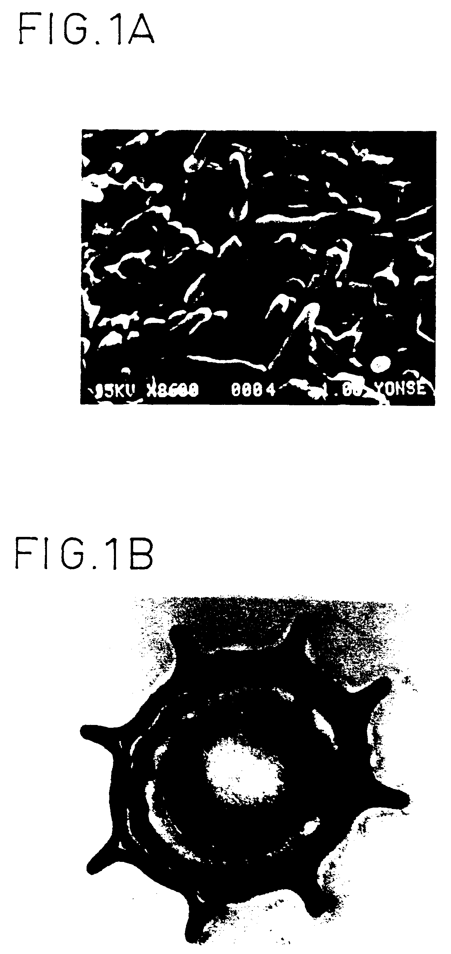

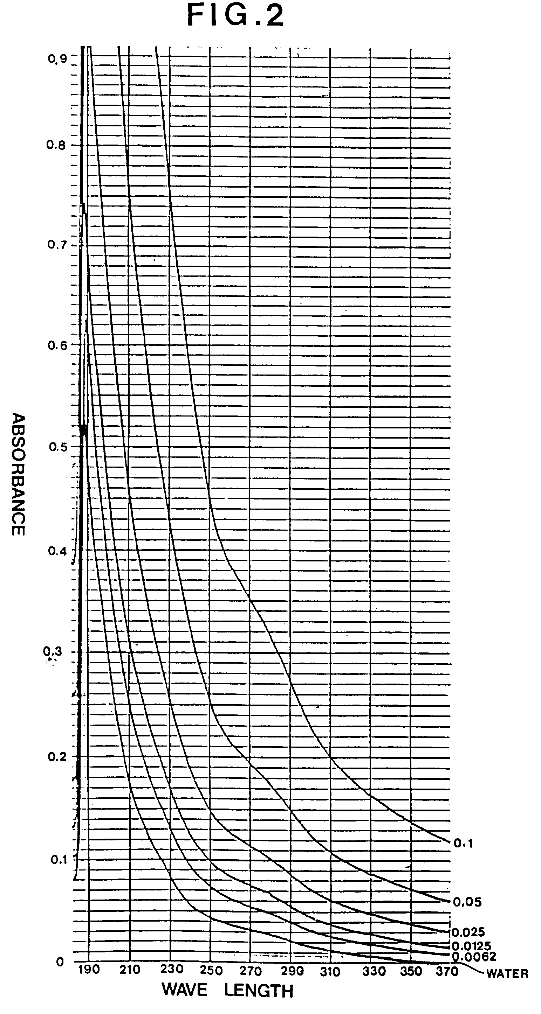

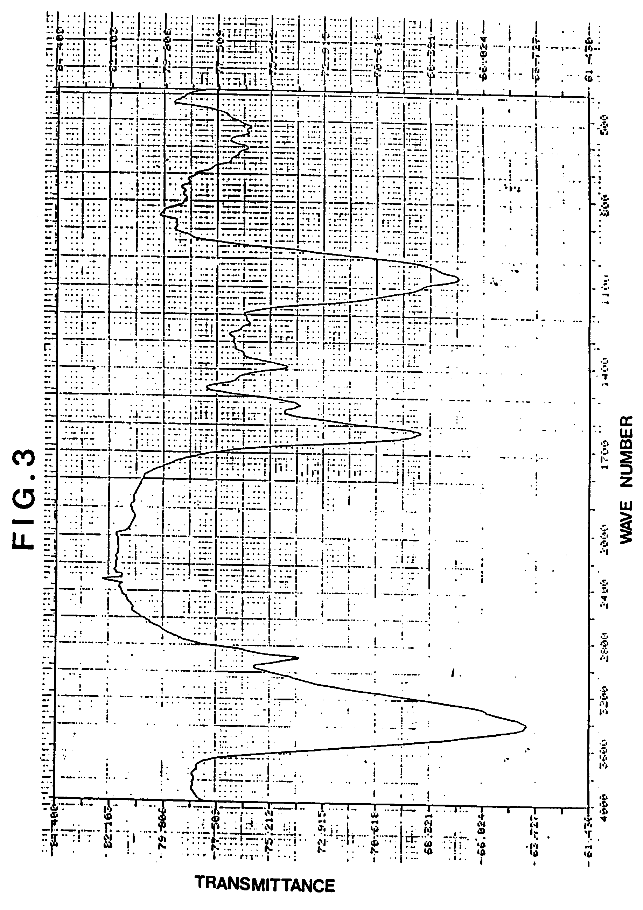

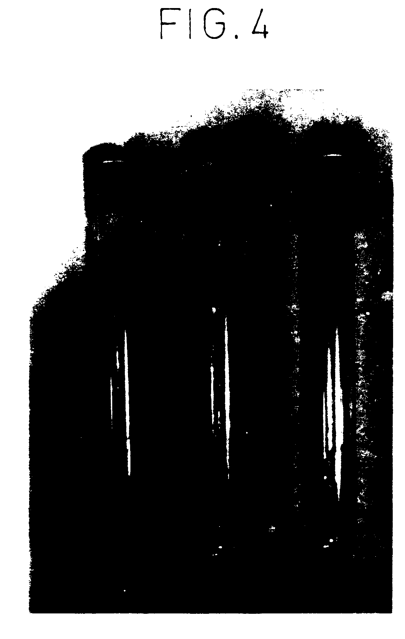

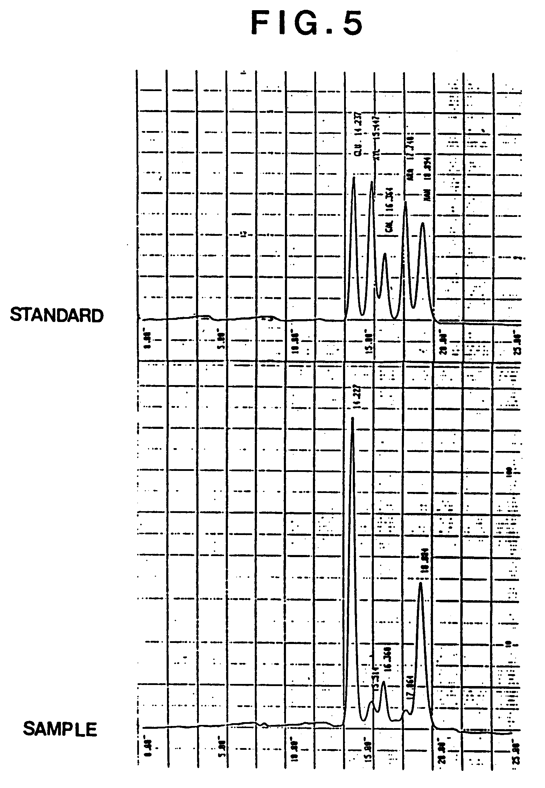

In figure 1, (a) is a picture for the external shape of Bacillus sp. Haitai 1 with an electron microscope and (b) is a picture of a transected spore of the strain with a scan microscope. Figure 2 is a graphical representation of the absorbance of different solutions (0,0062 to 0,1 %) of the roughly purified polysaccharide produced by Bacillus sp. Haitai 1, at 190-370 nm measured with a spectrophotometer. The IR spectrum of the polysaccharide of figure 2 is represented in figure 3. Figure 4 is a picture representing (a) a thermally treated cultivating tube of Bacillus sp. Haitai 1, (b) a thermally untreated cultivating tube of Bacillus sp. Haitai 1 and (c) a tube loaded with the culture medium before inoculating the strain. Polysaccharides of figure 2 are hydrolyzed by an acid, the constituent carbohydrates are analyzed by HPLC and the results are shown in figure 5.

### Detailed Description of the Invention

Bacillus sp. Haitai 1 of the present invention has the following bacteriological properties :
(1) They have the ability of spore formation.
(2) When cultivated in liquid, they produce the novel polysaccharides which have a thermal stability and form reversibly a gel by heat.
(3) They produce polysaccharide having the above character on agar plate.
(4) They grow in initial pH 3.3 - 7.9.

A normal medium comprising carbon source, nitrogen source and each mineral can be used as the medium of Bacillus sp. Haitai 1 characterized by the above properties. The carbon source may be carbohydrates such as, for example, glucose, galactose, mannose, fructose, xylose, sucrose, lactose, maltose or starch. The nitrogen source may be an organic nitrogen source such as corn steep liquor, peptone, yeast extract, dry yeast, soysauce or soybean meal and the mineral salts may be, for example, phosphate, calcium, manganese or sodium salts.

The culture temperature of Bacillus sp. Haitai 1 according to the present invention is 10°C - 40°C, preferably 25°C-37°C, and the initial pH is pH 3.3 - 7.9. As Bacillus sp. Haitai 1 is a facultative aerobic bacteria, the culture is preferably supplied with oxygen.

The method for separating Bacillus sp. Haitai 1 from soil is described in detail as follows.
About 2,000 soil samples were collected in all the country. A spoonful of soil sample is introduced into a sterilized test tube and treated at 80°C for 30 min. After being suspended by the addition of 3 ml of physiological saline solution, the solution is applied to the agar medium plate comprising 2% starch, 1 % soy bean meal, 0.2% KH₂PO₄, 0.2% NaCl, 0.1% MgSO₄.7H₂O and 0.2% CaCl₂. After incubation at 30°C for 2-3 days, the strains forming a viscous group are primarily screened.
These strains are inoculated in the liquid medium described above and cultivated at 30°C in a shaking incubator for 2-3 days. In order to collect, the viscous group, the culture is heated at 100°C for 5 minutes, and then cooled to room temperature to screen a strain producing a viscous polysaccharide.
The bacteriological properties of the screened strain are as follows :

### (1) Morphology

1. The results are observed by microscope
   1) Vegetative cell: Bacillus
   2) Motility: yes
   3) Size: 0.7 x 3.2-3.8»m
   4) Gram's stainability: Positive
   5) Spore: Star-like spore due to the formation of a patch and to the heavy spore coat (Figure 1 (b))
2. Growing states in various culture media
   1) Nutrient agar (Difco) plate culture: the strains grow hardly
   2) Nutrient broth (Difco) culture: the strains do not make film on the top
   3) Nutrient gelatin (Difco) culture: liquefaction is rapidly developed.
   4) Brain heart infusion (Difco) culture: growing states are fine.
   5) Growing states in a culture medium including Nacl:
      0% - growth
      2% - growth
      5% - difficult
      7% - difficult
      10% - difficult

### (2) Physiological properties

1. Growth temperature : 10°C-40°C
2. Optimum growth temperature: 27°C
3. Growth pH: 3.3-7.9
4. Optimum growth pH : 7.4
5. Attitude to oxygen: facultative aerobic
6. Decomposition of starch : Positive
7. Decomposition of casein : Positive
8. Decomposition of dextran : Negative
9. Decomposition of cellulose : Positive
10. Decomposition of xanthan : Positive
11. Decomposition of mannan : Positive
12. Generation of indole : Negative
13. Methylred test : Negative
14. Catalase test : Positive
15. Oxydase test : Positive
16. Urease test : Negative
17. Generation of dihydroxyacetone : Positive
18. Decomposition of tyrosine : Negative
19. VP test : Negative
20. Generation of phenylalanine diaminase : Negative
21. Generation of lecithinase : Negative
22. Reduction of nitrates : Positive
23. Hemolysis : Positive

### (3) Fermentation of carbohydrates

Each carbohydrate is added to the solution including 1% peptone, 0.2% KH₂PO₄, 0.2% NaCl, 0.05% MgSO₄, and 0.00625% Bromocrezol purple to provide a final concentration of 1%.
The reaction mixture is transferred to the test tube inserted Durham tube, sterilized, inoculated with the screened strain and incubated at 37°C incubator for 14 days while observing whether a gas and/or an acid are formed.

The results are shown in the following table.

| Carbohydrates | Formation of acid | Formation of gas |
|---|---|---|
| Arabinose | + | + |
| Cellobiose | + | + |
| Fructose | + | + |
| Galactose | + | + |
| Glucose | + | + |
| Glycerol | + | + |
| Inositol | - | - |
| Lactose | + | + |
| Maltose | + | + |
| Mannitol | + | + |
| Mannose | + | + |
| Melibiose | + | + |
| Raffinose | + | + |
| Salicine | + | + |
| Sorbitol | - | - |
| Sorbose | - | - |
| Starch | + | + |
| Sucrose | + | + |
| Xylose | + | + |

According to the above characters the strain of the present invention is classified according to Bergey's Manual of systematic bacteriology (1st Edition (1986)). The separated strain is identified as Bacillus. Considering the strain which has a star-like spore and produces an acid and gas to glucose, the strain may be included in Bacillus polymyxa. However, when it is considered that the strain strongly decomposes cellulose and grows below pH 5, the strain of the present invention is identified as a novel strain of Bacillus. Accordingly, the strain has been called Bacillus sp. Haitai 1, and deposited as an international deposit (under the Budapest Treaty on the International Recognition of the Deposit of Microorganisms for the Purposes of Patent Procedure) in Korean Culture Center of Microorganisms with the accession number of KCCM-10001 on July 1, 1990.

The preparation method of the novel polysaccharide produced by the strain according to the present invention is described as follows. First, cultivating the strain in the fermentation medium: the applicable medium may be a medium comprising a carbon source, a nitrogen source and mineral salts, suitable for growing the microorganism. The carbon source needed for cultivation may be, for example, starch, mannose, lactose, maltose, glucose, fructose or galactose. The nitrogen source may be for example corn steep liquor, peptone, dry yeast, soysauce or soybean meal, and the mineral salts may be phosphate, sodium, manganese or calcium salts.

According to the present invention, the strain is cultivated at pH 5.5-6.5 at a temperature of 25°C-37°C, generally for 2-3 days. During cultivation, the strain is preferably supplied with air as the strain is a facultative aerobic bacteria.
The microorganisms and insoluble material are then removed in order to purify the polysaccharide from the cultivating solution, as the polysaccharide subject of the present invention is included in the culture solution.
For example, three volumes of water are added to the cultivating solution to decrease the viscosity, the solution is then centrifuged to remove the microorganisms and insoluble material, and a solvent such as isopropyl alcohol is added to precipitate the polysaccharide. After being dried, the resulting precipitate is dissolved in water, and a solvent such as isopropyl alcohol is then added to reprecipitate the polysaccharide.

The above procedure is repeated several times and the resulting precipitate is dried under vacuum to obtain the purified polysaccharide produced by the strain.

The results of the analysis of the polysaccharide obtained by the above method are as follows :
(1) Compositions

| | |
|---|---|
| crude protein | 10.06 |
| ash | 4.02 |
| crude lipid | 0.43 |
| carbohydrates | 85.49 |
| UV spectrum (figure 2) λ max | 190nm |

(3) IR spectrum (figure 3)
(4) Solubility in a solvent
soluble in water and insoluble in organic solvents such as isopropyl alcohol, ethanol, methanol and acetone
(5) Coloured reaction
Sulfate reaction (to carbohydrates) : Positive
Molish's reaction (to carbohydrates) : Positive
Reaction of 3% FeCl₃ : Negative
Iodide reaction : Negative
Aniline - phthalate reaction : Negative
(6) Colour of the material : white
(7) Viscosity
a 1% solution of the polysaccharide is measured by SV-I cylinder of Haake viscometer. When treated by heat the viscosity is 2219 mpa.s ; without treatment, the viscosity is 1253 mpa.s.
(8) Atomic analysis

| | | |
|---|---|---|
| C : 33.6%, | H : 4.98%, | N : 1.2% |

(9) Thermal stability and gel forming capacity (figure 4)
(10) Analysis of the constituent carbohydrates
10 mg of the separated polysaccharide are dissolved in 1 ml of 2N H₂SO₄, hydrolyzed at 100°C for 6 hours and the obtained monosaccharides are analyzed by HPLC (model-Waters). As a result, the constituent carbohydrates are glucose, mannose, galactose, xylose and arabinose, and the molar ratio of glucose, galactose and mannose of them 1.5-7.3:1:0.9-4.8.

The polysaccharide according to the present invention has the capacity of water holding, film forming, and emulsifying, in addition to the above characters.
The polysaccharide of the present invention may be applied to various purposes either alone or mixed or with another polysaccharide in the industry of foods, cosmetics, oils and medicines.

The following Example further illustrates the present invention in detail.

### Example

Particular examples of the composition of the culture medium are specified in the following:

### Composition of culture medium

1. Culture medium A
   2% starch, 1% soybean meal, 0.1% KH₂PO₄, 0.1% NaCl, MgSo₄.7H₂O
   (sterilized at pH 5.8, 121°C for 15 minutes in vapor pressure)
2. Culture medium B
   1% Trypton, 1% NaCl, 0.5% yeast extract
   (sterilized at pH7.0, 121°C in vapor pressure)

25 ml of medium B are placed in a 100 ml Erlenmeyer flask, inoculated with Bacillus sp. Haitai 1 and cultivated at 230 rpm at 32°C for 16 hours. 25 ml of the above culture solution is inoculated in an Erlenmeyer flask including 175 ml of medium B and cultivated at 230 rpm at 32°C for 8 hours.
The culture solution is inoculated in a 7 l fermentation vessel including 3.5 l of medium A and cultivated at 32°C for 24 hours under 50% of dissolving oxygen. 7 l of deionized distilled water are added to 3.5 l of the culture solution. The solution is then stirred at 40°C and centrifuged at 8,000rpm for 30 minutes to remove the microorganisms. 5 volumes of isopropyl alcohol are added to the supernatant to precipitate the polysaccharide. The precipitation by isopropyl alcohol is repeated three times and the precipitate is dried under vacuum to obtain 22,43 g of crude polysaccharide. The yield is 32%.

The obtained polysaccharide has the above characteristics and its constituent carbohydrates are, after analysis, determined to be glucose, mannose, galactose, xylose and arabinose.

## Claims

1. A Bacillus sp. Haitai 1 strain as deposited at the KCCM under No. 10001.

2. A microorganism according to claim 1, capable of producing a polysaccharide, wherein said polysaccharide comprises glucose, mannose, galactose, xylose and arabinose and wherein the molar ratio of glucose, galactose and mannose in said polysaccharide is 1.5-7.3 : 1 : 0.9-4.8.

3. A process for the preparation of a polysaccharide, which comprises cultivating Bacillus sp. Haitai 1 (KCCM-10001) in a culture medium comprising a carbon source, a nitrogen source and mineral salts.

4. A polysaccharide obtainable from Bacillus sp. Haitai 1 (KCCM-10001), wherein said polysaccharide comprises glucose, mannose, galactose, xylose and arabinose, wherein the molar ratio of glucose, galactose and mannose in said polysaccharide is 1.5-7.3 : 1 : 0.9-4.8 .

5. A polysaccharide according to claim 4, wherein said polysaccharide is viscous, thermally stable, and has the capacities of film forming, water holding, emulsifying and reversibly forming a gel by a heat treatment.

## Patentansprüche

1. Bacillus sp. Haitai 1-Stamm, wie hinterlegt am KCCM unter der Hinterlegungs-Nr. 10001.

2. Mikroorganismus nach Anspruch 1, fähig zur Produktion eines Polysaccharids, worin das Polysaccharid Glucose, Mannose, Galactose, Xylose und Arabinose umfasst, und worin das Molverhältnis Glucose, Galactose und Mannose im besagten Polysaccharid 1,5-7,3:1:0,9-4,8 ist.

3. Verfahren zur Herstellung eines Polysaccharids, welches das Kultivieren von Bacillus sp. Haitai 1 (KCCM-10001) in einem Kulturmedium, enthaltend eine Kohlenstoffquelle, eine Stickstoffquelle und Mineralsalze, umfasst.

4. Polysaccharid, erhältlich aus Bacillus sp. Haitai 1 (KCCM-10001), worin das Polysaccharid Glucose, Mannose, Galactose, Xylose und Arabinose enthält, und worin das Molverhältnis von Glucose, Galactose, Mannose im besagten Polysaccharid 1,5-7,3:1:0,9-4,8 ist.

5. Polysaccharid nach Anspruch 4, worin das Polysaccharid viskos, thermisch stabil ist und filmbildende, Wasser zurückhaltende, emulgierende Eigenschaften hat und reversibel ein Gel durch Hitzebehandlung bildet.

## Revendications

1. Souche de Bacillus sp. Haitai 1 telle que déposée auprès de KCCM sous le n° 10001.

2. Micro-organisme selon la revendication 1 capable de produire un polysaccharide, ledit polysaccharide comprenant du glucose, du mannose, du galactose, du xylose et de l'arabinose et le rapport molaire du glucose, du galactose et du mannose dans ledit polysaccharide étant de 1,5 - 7,3 : 1:0,9-4,8.

3. Procédé de préparation d'un polysaccharide, lequel comprend la culture de Bacillus sp. Haitai 1 (KCCM-10001) dans un milieu de culture comprenant une source de carbone, une source d'azote et des sels minéraux.

4. Polysaccharide qu'il est possible d'obtenir à partir de Bacillus sp. Haitai 1 (KCCM-10001), ledit polysaccharide comprenant du glucose, du mannose, du galactose, du xylose et de l'arabinose, le rapport molaire du glucose, du galactose et du mannose dans ledit polysaccharide étant de 1,5 - 7,3 : 1 : 0,9 - 4,8.

5. Polysaccharide selon la revendication 4, ledit polysaccharide étant visqueux, stable à la chaleur et ayant des capacités filmogènes, de rétention d'eau, d'émulsification et de formation réversible d'un gel par un traitement thermique.
